# EUROPEAN PATENT APPLICATION

(11) **EP 4 610 653 A1**
(43) Date of publication of application: **03.09.2025**
(21) Application number: 23881949.4
(22) Date of filing: 27.10.2023
(51) Int. Cl.: G01N 33/543, G01N 21/64, G01N 21/76

(54) **METHOD FOR DETECTING ANALYTE IN SAMPLE AND IMMUNOASSAY TEST STRIP**

(30) Priority: 27.10.2022 CN 202211328431
(71) Applicant: Leadway (HK) Limited, Sheung Wan, Hong Kong (CN)
(72) Inventor: ZHENG, Jingxian, Hangzhou, Zhejiang 310030 (CN); LI, Senyuan, Hangzhou, Zhejiang 310030 (CN); MAO, Limin, Hangzhou, Zhejiang 310030 (CN)
(74) Representative: Schiweck Weinzierl Koch Patentanwälte Partnerschaft mbB
(86) International application number: PCT/CN2023/127047
(87) International publication number: WO 2024/088374

(57) **Abstract**

The present invention provides a method and an immunoassay test strip for detecting an analyte in a sample, wherein a label reagent and a capture reagent are used. The label reagent includes a first reagent marked by an analysis marker, and the first reagent is capable of specifically binding the analyte; the capture reagent is capable of capturing the first reagent marked by the analysis marker on a T line of the immunoassay test strip; a correction marker is coated on the T line, or is not coated on the T line but is capable of generating a correction signal on the T line during detection; and the signals generated by the correction marker and the analysis marker are mutually distinguishable. The detection reagent of the present invention can be used in the field of immunoassay such as chemiluminiscent immunoassay for qualitative and quantitative analysis of inflammatory markers, tumor markers, bone metabolism-series markers, sex hormone markers, thyroid function markers, infectious disease markers, hepatic fibrosis markers, diabetes markers, intestinal health-series markers, allergy markers, drug abuse, and other physiological indicators.

## Description

### TECHNICAL FIELD

The present invention belongs to the technical field of biological detection, and relates to a method and an immunoassay test strip for detecting an analyte in a sample.

### BACKGROUND

In recent years, the rapid diagnostic techniques and detection methods in immunology have made rapidly progress and have been widely applied to a series of fields such as medical inspection, pharmaceutical analysis, environmental analysis, food analysis, and biomedicine. With numerous advantages such as quickness, simplicity and convenience, accuracy, and stability, immunochromatographic technology now has become one of the commonly used clinical diagnostic techniques. Based on the principle of immunochromatography, a signal substance (hereafter referred to as a signal marker) for labeling an antibody or antigen is aggregated on the test line (T line) of an immunoassay test strip to generate a particular signal. The level of the signal is correlated to the concentration of an analyte (an antibody, antigen, or hapten) to be tested, thereby achieving the qualitative and/or quantitative detection of the analyte.

In quantitative immunochromatography based on optical detection, an immunoassay test strip is irradiated by light from a light source, and then a detector is utilized to collect an optical signal such as reflected light, transmission light, fluorescence, or phosphorescence generated on the T line and/or control line (C line) of the immunoassay test strip after being irradiated. Afterwards, the collected optical signal is transformed into an electric signal; and the level of the electric signal is correlated to the concentration of the analyte to be tested, thereby quantitatively detecting the concentration of the analyte. In addition, during the process of calculating the concentration of the analyte, the optical signal of the C line can be usually utilized to correct the optical signal of the T line, so as to eliminate or minimize the adverse effect on test results caused by the differences existing in the manufacturing process of the immunoassay test strip.

During the manufacturing process of the immunoassay test strip, a liquid scriber or a liquid sprayer is usually used to apply a T line solution and a C line solution onto the corresponding T and C lines of the immunoassay test strip. The former is to deposit the T line solution and the C line solution onto the detection pad surface of the immunoassay test strip with a liquid scribing head of the liquid scriber in a liquid spotting manner; the latter is to spray the T line solution and the C line solution onto the detection pad surface of the immunoassay test strip. However, whether for liquid spotting or spraying, due to a certain failure rate in the instrument itself during the manufacturing process, or due to manual operational errors or improper setting of instrument parameters , the solutions added onto the T lines and C lines of the same batch of immunoassay test strips may not be uniform, for example, the volume of the solutions added onto some T lines and C lines is too much, while the volume of the solutions added onto some other T lines and C lines are too little, thus resulting in inconsistency in the sizes of the stripes on the T lines and C lines of the same batch of immunoassay test strips; for example, the solutions added onto some T lines and C lines are not continuous, thereby leading to discontinuous stripes on the T lines and C lines. These can result in inconsistent signals generated on the T lines of the same batch of immunoassay test strips, which is likely to lead to detection errors.

Moreover, in the prior art, since the T line solution and the C line solution are not added onto the same line on the detection pad surface, variations in the amounts of the added T line solution and the added C line solution may occur asynchronously, thus leading to inaccuracies in the analyte concentration calculated based on a signal ratio: T/T' or T/(T+T') when using the optical signal of the C line to calibrate the optical signal of the T line.

### SUMMARY

To address the deficiencies of the prior art, the present invention provides an immunoassay test strip, a test kit, a test cassette, and a method for detecting an analyte in a sample. After the sample is added, a detection signal T and a correction signal T' can be generated on a T line of the immunoassay test strip, and the T signal is corrected via the T' signal to obtain a corrected T signal, and then a concentration of the analyte may be obtained accurately according to the corrected T signal.

In view of this, the present invention provides a detection reagent for use in an immunoassay test strip, including a label reagent and a capture reagent; the label reagent includes a first reagent marked by an analysis marker, and the first reagent is capable of specifically binding the analyte; the capture reagent is capable of capturing the first reagent marked by the analysis marker on the immunoassay test strip; the detection reagent further includes a correction marker, and the signals generated by the analysis marker and the correction marker are mutually distinguishable; the correction marker and the capture reagent are coated on a same position (e.g., the T line of the immunoassay test strip) of the immunoassay test strip, or the correction marker is not coated on the same position of the immunoassay test strip as the capture reagent, but is capable of generating a signal on the same position of the immunoassay test strip as the analysis marker during the detection.

In some cases, the immunoassay test strip includes a sample addition region and a detection pad; the sample addition region is located upstream of the detection pad, the detection pad is provided with a T line, and during detection, the label reagent is located in the sample addition region or between the sample addition region and the T line and after the sample is added to the sample addition region, the label reagent can move to the T line; the capture reagent is coated on the T line and is capable of capturing the first reagent marked by the analysis marker on the T line. The T line may be a line or a detection region located on the detection pad.

The present invention further provides a method for detecting an analyte in a sample, wherein the method includes the following steps: (1) providing an immunoassay test strip including a sample addition region and a detection pad, wherein the sample addition region is located upstream of the detection pad, the detection pad is provided with a T line, and during detection, the label reagent is located in the sample addition region or between the sample addition region and the T line and after the sample is added into the sample addition region, the label reagent can move to the T line; the label reagent includes a first reagent marked by an analysis marker, and the first reagent is capable of specifically binding the analyte; the T line is coated with a capture reagent capable of capturing the first reagent marked by the analysis marker on the T line; the correction marker is coated on the T line, or is not coated on the T line but is capable of generating a correction signal on the T line during detection; and the signals generated by the correction marker and the analysis marker are mutually distinguishable; (2) adding the sample into the sample addition region, so that the label reagent can move to the T line along with the sample and be captured by the capture reagent on the T line; (3) utilizing an analyzer to detect a detection signal T generated by the analysis marker on the T line and a correction signal T' generated by the correction marker on the T line; and (4) calculating a concentration of the analyte based on the signal T and the signal T'.

In some cases, the signal T is corrected by the signal T' to obtain a corrected signal, and a concentration of the analyte is calculated based on the corrected signal. In some cases, the corrected signal is a signal ratio: T/T' or T/(T+T').

The present invention further provides an immunoassay test strip for detecting an analyte in a sample, including a sample addition region and a detection pad, wherein the sample addition region is located upstream of the detection pad, the detection pad is provided with a T line, and during detection, the label reagent is located in the sample addition region or between the sample addition region and the T line; and after the sample is added into the sample addition region, the label reagent can move to the T line; the label reagent includes a first reagent marked by an analysis marker, and the first reagent is capable of specifically binding the analyte; the T line is coated with a capture reagent capable of capturing the first reagent marked by the analysis marker on the T line; a correction marker is coated on the T line, or is not coated on the T line but is capable of generating a correction signal on the T line during detection; and the signals generated by the correction marker and the analysis marker are mutually distinguishable.

In some cases, the detection pad is further provided with a C line; the label reagent further includes a reference reagent marked by a reference marker; a reference capture reagent coated on the C line is capable of capturing the reference reagent marked by the reference marker on the C line. In some cases, the reference reagent/reference capture reagent is selected from nonhuman antibody/anti-nonhuman antibody, biotin/streptavidin, DNP-BSA/anti-DNP antibody, and receptor/ligand. For example, the reference reagent/reference capture reagent may be selected from DNP-BSA/rabbit anti-DNP antibody.

In some cases, the reference marker is selected from a lanthanide element or a chelate thereof, a platinum/palladium porphyrin compound, a time-resolved luminescent microsphere, a colored luminescent microsphere, a colored colloidal particle, a magnetic nanoparticle and a luminescent compound. In some cases, the analysis marker and the reference marker are a time-resolved fluorescent microsphere, and the correction marker is a green fluorescent microsphere.

In some cases, the label reagent is coated on the immunoassay test strip, or added onto the immunoassay test strip during detection. In some cases, the immunoassay test strip includes a sample addition pad on which the sample addition region is located. In some cases, the label reagent is coated on the sample addition pad. In some cases, the immunoassay test strip includes a label pad, the sample addition region is located on the label pad, and the label reagent is coated on the label pad.

In some cases, the immunoassay test strip includes a sample addition pad and a label pad; the label pad is located between the sample addition pad and the detection pad; the sample addition region is located on the sample addition pad, and the label reagent is coated on the label pad.

In some cases, the analysis marker and the correction marker are selected from a lanthanide element or a chelate thereof, a platinum/palladium porphyrin compound, a time-resolved luminescent microsphere, a colored luminescent microsphere, a colored colloidal particle, a magnetic nanoparticle and a luminescent compound. In some cases, the analysis marker is a time-resolved fluorescent microsphere, and the correction marker is a green fluorescent microsphere. In some other cases, the analysis marker is a time-resolved fluorescent microsphere, and the correction marker is fluorescein isothiocyanate (FITC) or a conjugate of FITC and a carrier protein.

In some cases, the label reagent includes a second reagent that is marked with the correction marker, and the capture reagent is capable of capturing the second reagent marked with the correction marker on the T line.

In some cases, the T line is also coated with a quality control marker, and the signals generated by the quality control marker, the analysis marker and the correction marker are mutually distinguishable. In some cases, the quality control marker is selected from a lanthanide element or a chelate thereof, a platinum/palladium porphyrin compound, an up-conversion luminescent material, a time-resolved luminescent microsphere, a colored luminescent microsphere, a colored colloidal particle, a magnetic nanoparticle and a luminescent compound. For example, the quality control marker may be selected from FITC.

In some cases, the capture reagent includes a first component and a second component, wherein the first component is capable of capturing the first reagent marked by the analysis marker on the T line, and the second component is capable of capturing the second reagent marked by the correction marker on the T line. In some cases, the first component and the second component may be coupled together via a coupling reagent, and may be also present independently.

In some cases, the second reagent and the second component do not specifically bind the first reagent, the first component, and the analyte. In some cases, the reference reagent and the reference capture reagent do not specifically bind the first reagent, the first component, the second reagent, the second component, and the analyte.

In some cases, the capture reagent merely includes one substance (e.g., protein A, protein G); for example, the first reagent is mouse IgG and the first component is mouse anti-sheep IgG antibody, at this time, the capture reagent may be protein G which may not only capture the mouse IgG, but also capture the mouse anti-sheep IgG antibody.

In some cases, when the analyte is an antigen, the first reagent and the first component are antibodies specifically binding the analyte; or the first reagent is an antibody specifically binding the analyte (e.g., mouse IgG antibody specifically binding the analyte), and the first component is a secondary antibody specifically binding the first reagent (e.g., rabbit anti-mouse IgG antibody specifically binding mouse IgG antibody); when the analyte is an antibody, the first reagent and the first component are antigens specifically binding the analyte; or one of the first reagent and the first component is an antigen specifically binding the analyte, and the other one is a secondary antibody specifically binding the analyte, or when the first reagent is an antigen specifically binding the analyte (e.g., a receptor binding domain of SARS-CoV-2 spike protein), and the first component (e.g., human ACE protein) and the analyte (e.g., SARS-CoV-2 neutralizing antibody) competitively bind the first reagent; when the analyte is a hapten, the first reagent is an antibody specifically binding the analyte, and the first component is a conjugate of the analyte or its analogue and a carrier protein (e.g., BSA, KLH, etc.).

In some cases, the second reagent/second component is selected from nonhuman antibody/anti-nonhuman antibody, biotin/streptavidin, DNP-BSA/anti-DNP antibody, and receptor/ligand. In some cases, the second reagent/second component is sheep anti-chicken IgY antibody/chicken IgY antibody.

In some cases, the correction marker is coated on the T line; the capture reagent includes a first component capable of capturing the first reagent marked by the analysis marker. In some cases, the correction marker is coupled with the first component, or the correction marker exists independently of the first component.

The "capture" mentioned in the present invention refers that the capture reagent coated on the T line or the reference capture reagent coated on the C line may capture the first reagent marked by the analysis marker, the second reagent marked by the correction marker, or the reference reagent marked by the reference marker onto the T or C line, directly or indirectly or via a bridging structure. The "directly" refers that the first reagent marked by the analysis marker, the second reagent marked by the correction marker, or the reference reagent marked by the reference marker itself is captured onto the T or C line. The "indirectly" refers that a complex formed by specifically binding the first reagent marked by the analysis marker to the analyte, a complex formed by specifically binding the second reagent marked by the correction marker to a first non-analyte, or a complex formed by binding the reference reagent marked by the reference marker to a second non-analyte is captured onto the T or C line. The bridging structure is formed by specific binding of compounds such as biotin and streptavidin, for example, the reference capture reagent carries biotin and the reference reagent carries streptavidin, such that the reference capture reagent may capture the reference reagent marked by the reference marker on the C line via the specific binding between biotin and streptavidin.

Beneficial effects: (1) in the present invention, the correction signal (T' signal) generated by the T line is utilized to correct the detection signal (T signal) generated by the same T line; that is, the first component and the second component are coated on the T line at one time, so when the volume of the added T line solution is too much or too little, and the amounts of the first component and the second component are also too much or too little at the same time, so the correction of the T signal via the T' signal can effectively eliminate the difference that is caused by the asynchronous volume change of the T and C line solutions added during manufacturing a test cassette and that cannot be eliminated by correcting the T signal with a control signal (C signal) generated by the C line, thereby accurately determining the concentration of the analyte in a sample, and thus the present invention is obviously superior to the conventional method of correcting the T signal with the control signal (C signal) generated by the C line in detection precision; (2) in the present invention, when the first component coated on the T line carries a quality control marker, such that during manufacturing test cassettes, the T lines in a test strip plate are directly irradiated by a laser flashlight, then the amount and uniformity of the T line solution coated on the T lines of the test strip plate are determined according to the optical signal generated by the first component carrying the quality control marker coated on the T lines, so there is no need to cut the test strip plate and no need to add a clinical sample, an analyte calibration solution and a sample dilution solution; if the result is obviously inconsistent with the quality control requirement, it is unnecessary to cut the test strip plate, which can avoid further processing the test strip plate obviously inconsistent with the quality control requirement to produce a large number of unqualified immunoassay test strips at the source, thus reducing the manufacturing time and costs; in the meantime, when a test cassette or immunoassay test strip is subjected to spot check, there is likewise no need to add a clinical sample, an analyte calibration solution and a sample dilution, but the T line of the test cassette is directly irradiated by light from the light source of an analyzer; and then it can be determined whether some batch of test cassettes meets the quality control requirement according to the fluorescent signal generated by the T line, thus improving the production efficiency, shortening the production cycle, and saving production costs.

### BRIEF DESCRIPTION OF THE DRAWINGS

FIG 1 is a breakdown schematic diagram of a test cassette used in the present invention;
FIG 2 is a schematic diagram showing an immunoassay test strip in the test cassette used in the present invention;
FIG 3 is a stereogram of an analyzer used in the present invention;
FIG 4 is a schematic diagram showing an internal circuit of an analyzer used in the present invention;
FIG 5 shows pictures of a detection pad captured by a camera device after adding a T line solution to the detection pad and before drying; and
FIG 6 shows pictures of a detection pad captured by a camera device after adding a T line solution to the detection pad and drying.

### DETAILED DESCRIPTION OF EMBODIMENTS

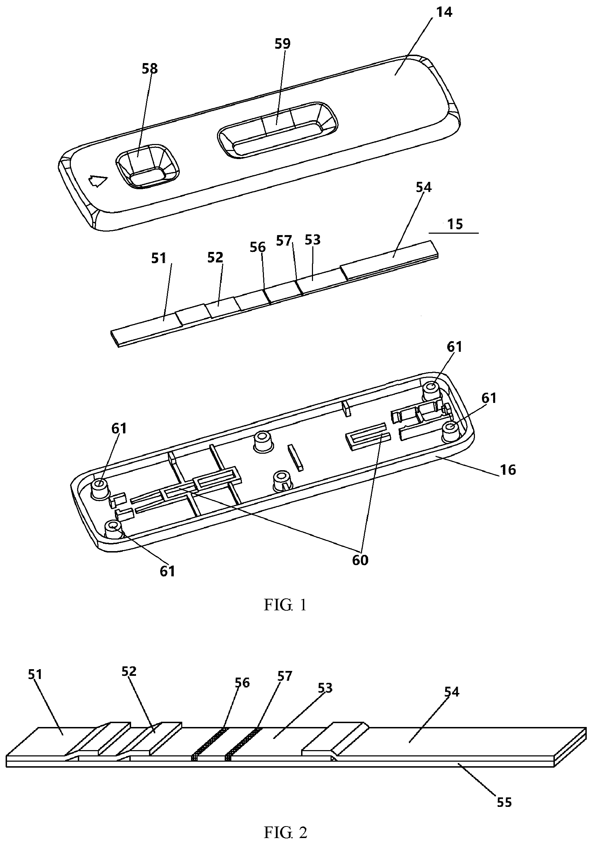

As shown in FIGS. 1-2, a test cassette 5 includes an immunoassay test strip 15, a cover 14, and a base 16. The used immunoassay test strip 15 is an immunochromatography test strip, including a sample addition pad 51, a label pad 52, a detection pad 53, and a sample absorption pad 54 that are connected sequentially in an overlapping arrangement. The detection pad 53 is made of nitrocellulose, glass fiber, polyether sulfone, nylon, and other materials, for example, the detection pad 53 is a nitrocellulose membrane. The detection pad 53 is provided with a test line (T line) 56 and a control line (C line) 57. The sample addition pad 51 is made of a water-absorbing material, and may be selected from glass fiber or non-woven fabrics. The label pad 52 is made of a water-absorbing material, and may be selected from polyester film, glass fiber or non-woven fabrics. The sample absorption pad 54 is disposed on a bottom supporting layer 55 and is made of a hydrophilic material; for example, the sample absorption pad 54 is filter paper. The number of the test lines disposed on the detection pad 53 may be adjusted according to the practical requirements.

The immunoassay test strip 15 further includes the bottom supporting layer 55 which is made of polyvinyl chloride and other common hydrophobic materials, to ensure that a sample may not permeate out of the bottom supporting layer 55. The sample addition pad 51, the label pad 52, the detection pad 53, and the sample absorption pad 54 are disposed on the bottom supporting layer 55. One end of the label pad 52 partially overlaps the sample addition pad 51, and the other end of the label pad 52 partially overlaps the detection pad 53, and one end of the sample absorption pad 54 partially overlaps the detection pad 53. In some cases, an overlapping region between any two adjacent pads is 0.5-5 mm in length.

The immunoassay test strip 15 is located within a shell formed via the assembly of the cover 14 and the base 16 via ultrasonic welding, buckling, or glue bonding. In some cases, the cover 14 and the base 16 are selected from plastic materials. A test strip groove 60 is disposed in the middle of the base 16 for accommodating the immunoassay test strip 15. In some cases, the cover 14 is provided with a plurality of buckles (not shown in the figures) extending downward; the base 16 is provided with a plurality of slots 61 extending upward; the buckles disposed in the cover 14 are one-to-one corresponding to the slots 61 disposed in the base 16 such that the cover 14 and the base 16 can make the immunoassay test strip 15 to be fixed in the test strip groove 60 when the cover 14, the immunoassay test strip 15, and the base 16 are assembled together.

The cover 14 is further provided with a sample inlet 58 and an observation window 59. The sample enters into the sample addition pad 51 below the sample inlet 58 via the sample inlet 58; under the action of capillary, the sample migrates to the sample absorption pad 54 along the length direction of the immunoassay test strip 15. The observation window 59 is disposed over the test line 56 and the control line 57 of the detection pad 53. The light from the light source of an analyzer can irradiate on the test line 56 and the control line 57 of the immunoassay test strip 15 via the transparent or semitransparent observation window 59. After being irradiated by the light from the light source, the optical signals generated by the test line 56 and/or the control line 57 may be output to a detector of the analyzer.

Depending on the type of the analyte to be tested (e.g., antigen, antibody, or hapten) and the immunoassay principle (double-antigen sandwich immunoassay, double-antibody sandwich immunoassay, competitive immunoassay, indirect immunoassay, capture immunoassay), there will be variations in the substances coated on the label pad 52 and the test line 56. Taking calprotectin as the analyte to be tested and the double-antibody sandwich immunoassay as the immunoassay principle for illustration, wherein fecal calprotectin serves as a biomarker for evaluating inflammatory bowel disease (IBD) and colorectal cancer. The label pad 52 is coated with the first reagent marked by the analysis marker (e.g., the analysis marker is a time-resolved fluorescent microsphere, and the first reagent is an anti-calprotectin detection antibody), the second reagent marked by the correction marker (e.g., the correction marker is a green fluorescent microsphere, and the second reagent is a sheep anti-chicken IgY antibody), and the reference reagent marked by the reference marker (e.g., the reference marker is a time-resolved fluorescent microsphere, and the reference reagent is DNP-BSA). The test line 56 is coated with a first component (e.g., an anti-calprotectin capture antibody) and a second component (e.g., a chicken IgY antibody). The first reagent and the first component specifically bind calprotectin in a clinical sample, so after the clinical sample is added to the sample addition pad 51, the clinical sample flows along the length direction of the immunoassay test strip 15. When the clinical sample reaches the label pad 52, the first reagent marked by the analysis marker specifically binds the calprotectin (if exists) in the clinical sample. The formed analysis marker-first reagent-calprotectin complex continues to flow and specifically binds the first component on the test line 56, and thus is captured on test line 56. Meanwhile, the second component on the test line 56 can capture the second reagent marked by the correction marker or a complex formed by the specific binding of the second reagent marked by the correction marker to a first non-analyte on the test line 56. After being irradiated by the light from a light source, the analysis marker captured on the test line 56 generates a detection signal T correlated to the calprotectin concentration, and the correction marker captured on the test line 56 generates a correction signal T'. In the meantime, the reference reagent marked by the reference marker continues to flow, and when it flows to the control line 57, the reference capture reagent (e.g., a rabbit anti-DNP antibody) coated on the control line 57 can capture the reference reagent marked by the reference marker or a complex formed by the specific binding of the reference reagent marked by the reference marker to a second non-analyte onto the control line 57. After being irradiated by the light from a light source, the reference marker captured on the control line 57 generates a control signal. The generated detection signal T, the correction signal T', and the control signal may be reflected light, transmission light, fluorescence, phosphorescence, and other optical signals generated by the T line and C line after being irradiated by the light from a light source. The reference marker may be the same as, or different from the analysis marker. The analysis marker and the correction marker are different and optical signals generated by the two may be distinguished by an analyzer without interfering each other.

The sample applied via the sample inlet 58 firstly enter into the sample addition region of the immunoassay test strip 15; the sample addition region is located upstream of the detection pad 53 and located on the sample addition pad 51. Even though the immunoassay test strip 15 as shown in FIGS. 1-2 contains the sample addition pad 51 and the label pad 52, but the sample addition pad 51 may be also absent according to the requirements and at this time, the sample addition region may be located on the label pad 52. In addition, the label pad 52 may be also absent and at this time, the reagent originally coated on the label pad 52 is preserved in a test tube in a form of liquid or solid; during detection, the sample is added to the test tube to form a mixture, and then the formed mixture is applied to the sample addition region of the immunoassay test strip 15 via the sample inlet 58, and moves towards the T line and C line along the length direction of the immunoassay test strip 15.

In some cases, the second component on the test line 56 can directly capture the second reagent marked by the correction marker onto the test line 56; the reference reagent marked by a reference marker is directly captured onto the control line 57 by the reference capture reagent coated on the control line 57. At this time, the second reagent and second component as well as the reference reagent and the reference capture reagent can be selected from any one of the following combinations: nonhuman antibody/anti-nonhuman antibody (e.g., rabbit IgG antibody/sheep anti-rabbit IgG antibody, and chicken IgY antibody/sheep anti-chicken IgY antibody), biotin/streptavidin, DNP-BSA/anti-DNP antibody, and receptor/ligand, etc. For example, the second reagent and second component are selected from chicken IgY antibody/sheep anti-chicken IgY antibody; the reference reagent and reference capture reagent are selected from DNP-BSA/anti-DNP antibody.

Each of the first non-analyte and the second non-analyte mentioned in the present invention refers to a substance that doesn't affect the detection of the analyte to be tested and the two are free of mutual effect with each other during detection. These non-analytes may be present or absent in the sample; if it is absent in the sample, it may be added to the sample or the immunoassay test strip in advance. Each of the first non-analyte and the second non-analyte may be a substance that is absent or may be ignored in quantity in the sample such that the correction signal and the control signal are not affected by the clinical sample. Each of the first non-analyte and the second non-analyte may be also a substance that is present in a sample at a higher level. For example, when the analyte to be tested is calprotectin, the first non-analyte and the second non-analyte in the present invention may be human IgG and human IgM present in the clinical sample, respectively; at this time, the second reagent and the reference reagent may be a rabbit anti-human IgG antibody specifically binding human IgG and a rabbit anti-human IgM antibody specifically binding human IgM, respectively; the second component coated on the T line is a sheep anti-rabbit IgG antibody specifically binding the rabbit anti-human IgG antibody; and the reference capture reagent coated on the control line 57 is a sheep anti-rabbit IgM antibody specifically binding the rabbit anti-human IgM antibody.

The analysis marker, the correction marker, and the reference marker may be selected from a time-resolved luminescent marker, a colored luminescent microsphere, a colored colloidal particle (e.g., latex, colloidal gold, colloidal carbon, and colloidal selenium), a magnetic nanoparticle, and a luminescent compound. The analysis marker and the reference marker are preferably a time-resolved luminescent marker. The time-resolved luminescent marker has a time-delayed luminescence property, that is, when the excitation light generated by a light source is turned off, the luminescent marker can still continuously generate light for a certain period of time. The time-resolved luminescent marker may be present in a form of molecule, called a time-resolved luminescent molecule, and may be selected from a lanthanide element such as Sm(III), Dy(III), Eu(III), Tb(III) and its chelate, a platinum/palladium porphyrin compound after being excited to generate phosphorescence, and an up-conversion luminescent material. A proper lanthanide chelate is N-(p-isothiocyanobenzene)-diethylenetriamine tetraacetic acid-Eu³⁺. The time-resolved luminescent marker may be also present in another form: a time-resolved luminescent microsphere, that is, the time-resolved luminescent molecule is encapsulated in or on the surface of a natural or synthetic microsphere or microbead. Each time-resolved luminescent microsphere can encapsulate tens of thousands of time-resolved luminescent molecules to effectively improve the detection sensitivity; hence, the time-resolved luminescent marker is preferably a time-resolved luminescent microsphere. The time-resolved luminescent microsphere after being excited to generate fluorescence is called a time-resolved fluorescent microsphere.

The colored luminescent microsphere refers to a microsphere or microbead whose surface or inner part is encapsulated by a luminous compound such as a quantum dot and a fluorescent dye; it may generate optical signals without a time-delayed luminescence property after being irradiated by a proper wavelength of excitation light. The colored luminescent microsphere may be selected from a green fluorescent microsphere, a blue fluorescent microsphere, a red fluorescent microsphere, a yellow fluorescent microsphere, and a colorful fluorescent microsphere (generating many specific colors of fluorescence). The colored colloidal particle refers to a colloidal particle that generates colored aggregates after aggregating on the T line and/or C line during immunochromatographic reaction, and may be selected from latex, colloidal gold, colloidal carbon, and colloidal selenium.

The C line in the present invention is not necessary; when it is configured, the control signal generated by the C line is useful in indicating whether the added clinical sample flows to the sample absorption pad 54. The control signal can be not only a light signal, but also a color signal, for example, colored aggregates are generated by the colored colloidal particles after aggregating on the control line 57. When the color signal is observed to appear on the control line 57, it can determine whether the added clinical sample flows into the sample absorption pad 54 or not. Therefore, there is no need to collect the reflected light signal generated on the C line after irradiation of a light source.

The luminescent compound in the present invention refers to a substance capable of generating light without a delayed luminescent property under the irradiation of a proper wavelength of excitation light, and may be selected from a quantum dot, fluorescein and derivatives thereof, for example FITC; a fluorescent protein capable of generating fluorescence after being excited and improved variants thereof, e.g., a green fluorescent protein, a red fluorescent protein, a blue fluorescent protein, a yellow fluorescent protein, an orange fluorescent protein, etc.; a chemical luminescent marker capable of being selected from luminol, isoluminol, and derivatives thereof, 1,2-dioxethane derivatives (including common AMPPD, CSPD, CDP, CDP-Star as well as PPD, Lumi-Phos, and Lumi-Plus from Lumigen, etc.), and an acridinium ester or acridine sulfonamide. The luminescent compound may be coupled to an antibody or antigen or a hapten-carrier protein conjugate via a conventional chemical coupling reagent.

Whether for the time-resolved luminescent microsphere or the colored luminescent microsphere, the surface thereof usually carries a radical such as hydroxyl, carboxyl, amino, formyl, and sulfonyl; it may be coupled to an antibody or antigen or a hapten-carrier protein conjugate via a conventional chemical coupling reagent, or coupled to an antibody or antigen or a hapten-carrier protein conjugate via a bridging structure. In some cases, the time-resolved luminescent microsphere has a size of 20 nm-100 µm; and the colored luminescent microsphere has a size of 100 nm-100 µm.

When there exists one or more analytes in a test sample, based on the different immunoassay principles, the intensity of the detection signal generated by the analysis marker on the T line is positively or negatively correlated to the concentration of the analyte; except the competitive immunoassay, the intensity of the detection signal generated by the analysis marker on the T line is positively correlated to the concentration of the analyte. However, no matter which kind of immunoassay principle is adopted, the control line 57 should generate a control signal; failure to produce a control signal indicates a problem, or failure, in the immunoassay test strip.

The clinical sample in the present invention may be selected from serum, plasma, whole blood, cerebrospinal fluid, urine, bronchoalveolar lavage fluid, nasopharyngeal swabs, sputum, faces, skin lesion sample (including a swab of exudates from rashes/pock pustule; pock pustule fluid, acne scab, etc.) etc. Based on the differences of the type of a clinical sample and the analyte, some clinical samples are pretreated (e.g., lysed by a lysis solution to release an analyte to be tested) before detection, and then added to the test cassette 5 for detection.

The analyte to be tested in the present invention includes an antigen or antibody, even a hapten, e.g., inflammatory marker, a heart failure marker, a tumor marker, a bone metabolism-series marker, a sex hormone marker, a thyroid function marker, an infectious disease marker, a diabetes marker, a hepatic fibrosis marker, an allergy marker, an intestinal health-series marker, drug abuse, etc.

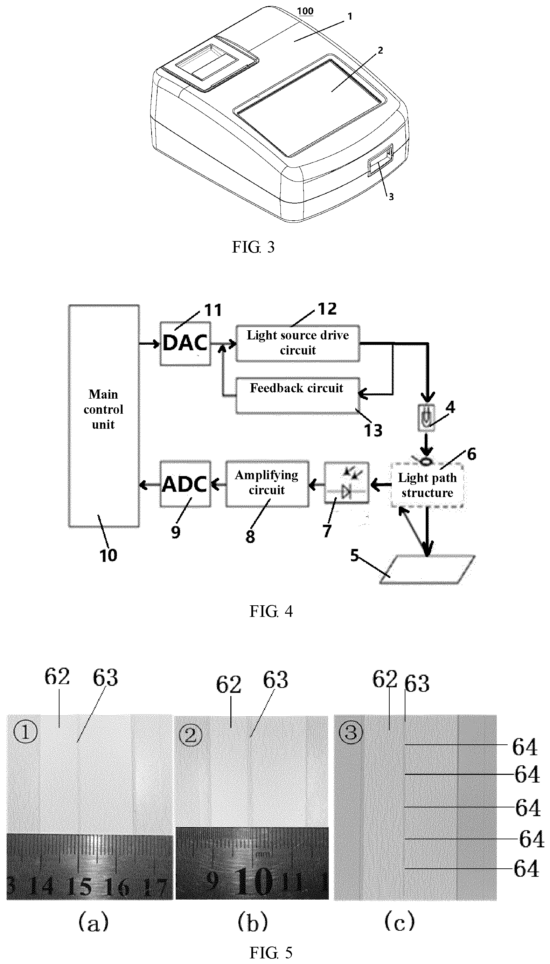

During detection, an analyzer is used to detect the test cassette 5. As shown in FIGS. 3-4, the analyzer 100 includes a shell 1, a display 2, and a test cassette insertion port 3. The test cassette 5 is connected to the analyzer 100 via the test cassette insertion port 3. The clinical sample is added to the test cassette 5 and is subjected to reaction for a period of time outside the analyzer 100, and then inserted into the analyzer 100 for detection; or the clinical sample is added to the test cassette 5 and then immediately inserted into the analyzer 100, then subjected to reaction in the analyzer 100 for a period of time for detection. The analyzer 100 further includes an optical system, an amplifying circuit 8, an analog-to-digital conversion chip (ADC) 9, a main control unit 10, and a digital-to-analog conversion chip (DAC) 11, which are located within the shell 1. The optical system includes a light source 4, a light path structure 6, a detector 7, a light source drive circuit 12, and a feedback circuit 13. The optical system may further be equipped with a narrow band filter or optical grating and a series of other optical devices according to the requirements. The light source 4 may be selected from a light emitting diode (LED), a flash lamp, and other proper light sources, and preferably LED. The illumination of the light source 4 may be continuous or impulse-type. The detector 7 may be selected from a photomultiplier tube, a photodiode (PD), a charged coupled device, a charge injection detector or CMOS photosensitive element, etc., and preferably, PD, e.g., a silicon photodiode. A plurality of the detectors 7 need to be configured according to the requirements. The analyzer may be also selected from commercially available analyzers.

The main control unit 10 includes a microprocessor; the control signal applied by the microprocessor is subjected to digital-to-analogue conversion via the DAC 11, to provide a light source current control signal to the light source drive circuit 12 such that the light source 4 works in a status of constant current after feedback through the feedback circuit 13. During scanning the test cassette 5 via the optical system, the light generated by the light source 4 passes through the light path structure 6 (e.g., optical fiber) and irradiates on the test cassette 5 on which a sample to be tested is added. After the sample is added for reaction for a period of time, optical signals are generated on the test line (also called T line) 56 and the control line (also called C line) 57 of the test cassette 5 after irradiation. The generated optical signals are output to the detector 7 through the light path structure 6 (e.g., an optical fiber, preferably, it is different from the optical fiber through which the light generated by the light source 4 passes), and are converted into current signals through the detector 7. The generated current signals are modulated and converted to a suitable voltage range by the amplifying circuit 8, and then subjected to analog-to-digital conversion by the ADC 9, and transferred to the main control unit 10. After the analog-to-digital conversion of the electric signals received, they can be calculated by the microprocessor in the main control unit 10, thus determining the presence or concentration of the analyte in the sample to be tested.

### Embodiment 1: Manufacturing the first type of the immunoassay test strip and test cassette in the present invention

A method of manufacturing an immunoassay test strip and a test cassette for use in the quantitative determination of calprotectin in faces includes the following steps:
A. Antibody preparation: an antigen was utilized to immunize a mouse, a rat, a rabbit and other animals, or hybridoma cells were counted to screen a pair of monoclonal antibodies or polyclonal antibodies for detecting calprotectin; a pair of commercialized anti-calprotectin antibodies (one was an anti-calprotectin capture antibody and the other was an anti-calprotectin detection antibody) could also be selected. Taking a pair of commercialized anti-calprotectin antibodies (Medix Biochemica, Art. No.: 100460 and 100618) as an example for illustration. A chicken IgY antibody, a sheep anti-chicken IgY antibody, and a rabbit anti-DNP antibody were self-prepared or purchased from the market.
B. Liquid spotting on the detection pad:
   The anti-calprotectin capture antibody and chicken IgY antibody were added to 0.02M phosphate buffer solution (pH 7.2) to obtain a T line solution; the anti-calprotectin capture antibody and the chicken IgY antibody in the T line solution had a concentration of 0.8 mg/ml and 0.4 mg/ml, respectively. The rabbit anti-DNP antibody was added to 0.02 M phosphate buffer solution (pH 7.2) to obtain a C line solution; the rabbit anti-DNP antibody in the C line solution had a concentration of 0.l mg/ml. Then, the T line solution and C line solution were coated on a nitrocellulose membrane having a width of 2.5 cm (as the detection pad) by a quantitative liquid spotting device (AUTOKUN continuous membrane-scribing machine/membrane-spotting machine HGS101, Hangzhou Fenghang Technology Co., Ltd.) in an amount of 1 µl/cm and at an interval of 0.8 cm, to form a T line and a C line, respectively, and then the membrane was dried for 18h at 45°C, and sealed with a desiccant for future use.
C. Preparation of fluorescent microspheres:
   Selection of Fluorescent microspheres: a time-resolved fluorescent microsphere having a diameter of 100 nm-400 nm (Suzhou Vdo Biotech Co., Ltd., Art. No.: FT0200CA) was selected, wherein an ionic lanthanide marker was encapsulated within the fluorescent microsphere and a carboxyl was carried on the surface of the fluorescent microsphere; for the fluorescent microsphere, the excitation wavelength was 360 nm and the emission wavelength was 615 nm. A green fluorescent microsphere having a diameter of 100 nm-400 nm (Suzhou Vdo Biotech Co., Ltd., Art. No.: FG0300CA) was selected, wherein a fluorescent dye was encapsulated within the fluorescent microsphere and a carboxyl was carried on the surface of the fluorescent microsphere; for the fluorescent microsphere, the excitation wavelength was 488 nm and the emission wavelength was 520 nm.

Preparation of MES buffer solution: MES sodium salt was added to pure water and mixed well such that MES sodium salt had a concentration of 1.066% (w/v), and then the mixture was filtered and sterilized by a 0.22 µm microporous filter membrane, and preserved at 4°C for future use.

Preparation of a preservation buffer solution: 50 mM Tris-HCl preservation buffer solution (pH 8.0) containing 20% sucrose (w/v), 5% trehalose (w/v), 2% Tween-20 (v/v), 0.5% PVP (w/v), and 0.5% Casein-Na(w/v) was used, filtered and sterilized by a 0.22 µm microporous filter membrane, and preserved at 4°C for future use.

Preparation of a time-resolved fluorescent microsphere coupled with the anti-calprotectin detection antibody (hereafter referred to as the TRF detection microsphere): the fluorescent microsphere was washed by the MES buffer solution, and carbodiimide (EDC) and N-hydroxysuccinimide (NHS) were added to achieve final concentrations of 0.4 mg/ml and 0.1 mg/ml, respectively. The resulting product reacted for 20 min at room temperature to activate the time-resolved fluorescent microsphere; after the time-resolved fluorescent microsphere was fully washed by the MES buffer solution, the anti-calprotectin detection antibody was added in a mass ratio of 1 mg:0.1 mg, and reacted for 1.5 h at room temperature, and then fully washed by the MES buffer solution. Then, 20 µl ethanolamine solution (10% BSA (w/v), 0.05M Tris-HCl (pH 7.9-8.1)) was added such that the final concentration of the ethanolamine was 2% (v/v). The resulting product reacted for 0.5 h at room temperature, and then fully washed by the MES buffer solution; then, 0.05M pH 8.0 Tris-HCl containing 10% BSA (w/v) was added for blocking for 1 h at room temperature. The time-resolved fluorescent microsphere was washed by the MES buffer solution, and then redissolved with the preservation buffer solution such that the final concentration of the time-resolved fluorescent microsphere was 0.2% g/ml, and preserved at 4°C for future use. According to the same method of preparing the TRF detection microsphere, a time-resolved fluorescent microsphere coupled with DNP-BSA and a green fluorescent microsphere coupled with a sheep anti-chicken IgY antibody were prepared, respectively. Moreover, the preservation buffer solution was utilized such that the time-resolved fluorescent microsphere coupled with DNP-BSA and the green fluorescent microsphere coupled with the sheep anti-chicken IgY antibody all had a final concentration of 0.2% g/ml, and then preserved at 4°C for future use. Finally, the time-resolved fluorescent microsphere coupled with the anti-calprotectin detection antibody, the time-resolved fluorescent microsphere coupled with DNP-BSA, the green fluorescent microsphere coupled with the sheep anti-chicken IgY antibody were mixed fully with the preservation buffer solution in a ratio of 5:2:5:8 to obtain a microsphere mixture.

### D. Spraying and drying of the fluorescent microsphere:

The prepared microsphere mixture was uniformly sprayed on a label pad (glass fiber) having a width of 1.0 cm in an amount of 4 µl/cm by a dedicated liquid scribing head of the AUTOKUN continuous membrane-scribing machine/membrane-spotting machine HGS101, dried for 18 h at 45°C, and sealed with a desiccant for future use.

### E. Treatment of the sample pad:

A sample pad having a width of 2.5 cm was placed into a sample pad treatment solution and soaked for 1 h, and then taken out and dried at 37°C overnight (12-24 h). The sample pad treatment solution was a buffer solution having pH 8.6 and containing 0.05% sodium tetraborate (w/v), 0.01% BSA (w/v), 0.5% ethylene diamine tetraacetic acid disodium (w/v), and 0.5% Tween-20 (v/v).

### F. Assembly and cutting of the test strip plate:

Assembly of the test strip plate: the 2.5 cm-wide nitrocellulose membrane, the 1.0 cm-wide label pad, the 2.5 cm-wide sample pad, and the 2.8 cm-wide absorbent paper (as the sample absorption pad) were assembled on a 8 cm-long plastic bottom plate (as the bottom supporting layer) manually or via a machine such that the sample pad, the label pad, the nitrocellulose membrane, and the absorbent paper were sequentially overlapped by 2 mm to form a test strip plate.

Cutting of the test strip plate: the formed test strip plate was cut into a single-use immunoassay test strip having a width of 0.4 cm by an AUTOKUN HGS201 strip cutting machine.

### G Assembly of the test cassette:

A single-use immunoassay test strip was placed into a slot of a plastic base, and covered with a cover; the base and the cover were tightened by a cassette pressing machine or manually to ensure that the whole immunoassay test strip is in a strained state; and the immunoassay test strip was placed with a desiccant, sealed at room temperature for future use.

After the test cassette was assembled, it may be placed into a test kit containing a desiccant and a user manual. In addition, the test kit may further include a sampling tube, a test tube with a lysis solution, a test tube with a sample dilution solution, a sampling swab, etc. according to the requirements.

### Embodiment 2: Manufacturing the second type of the immunoassay test strip and test cassette in the present invention as well as the test method

The immunoassay test strip and test cassette manufactured in the first scheme of Embodiment 2 merely differed from the Embodiment 1 in the following: the anti-calprotectin capture antibody in Embodiment 1 was replaced by a FITC-anti-calprotectin capture antibody conjugate such that the spotting amount and uniformity of the T line were used for quality control. The preparation method of the FITC-anti-calprotectin capture antibody conjugate: FITC was activated by NHS and then coupled with the anti-calprotectin capture antibody; the resulting FITC-anti-calprotectin capture antibody conjugate was diluted by the preservation buffer solution in Embodiment 1 to obtain a final concentration of 0.8 mg/mL. During the process of manufacturing a batch of test cassettes, before cutting the test strip plate, there was no need to add a clinical sample, a calprotectin calibration solution, or a sample dilution solution, and T lines were directly irradiated by a laser flashlight. According to the fluorescence generated by the FITC-anti-calprotectin capture antibody conjugate coated on the T lines, the amount and uniformity of the T line solution coated on the T lines of the test strip plate were determined qualitatively, and if the two parameters failed to meet the quality control requirement, there was no need to cut the test strip plate, thus saving the manufacturing time and costs.

The single-use immunoassay test strips cut from the test strip plate were assembled to obtain a batch of test cassettes. During the spot check of the test cassettes, the clinical sample, the calprotectin calibration solution, or the sample dilution solution was also not required; the T lines of the test cassettes used for the spot check were directly irradiated by an excitation light with a wavelength of 494 nm generated by a light source of a dry-type fluorescence immunoanalyzer FIC-Q100N (Suzhou Helmen Precision Instruments Co., Ltd.), and the emission light with a wavelength of 520 nm generated by the FITC-anti-calprotectin capture antibody conjugate coated on the T lines was counted to obtain fluorescent signals from the T lines. The fluorescent signals were used to quantitatively determine the amount and uniformity of the T line solution coated on the T lines of the single-use immunoassay test strips, thereby determining whether this batch of test cassettes met the quality control requirements.

The second scheme of Embodiment 2 differed from the first scheme in the following: the label pad was merely coated with the time-resolved fluorescent microsphere coupled with the anti-calprotectin detection antibody, the time-resolved fluorescent microsphere coupled with DNP-BSA, and the preservation buffer solution, but was free of the green fluorescent microsphere coupled with the sheep anti-chicken IgY antibody; during liquid spotting on the detection pad, T line was merely coated with the FITC-anti-calprotectin capture antibody conjugate but free of the chicken IgY antibody. After the clinical sample was added for reaction for a period of time, the time-resolved fluorescent signal (T signal) generated by the time-resolved fluorescent microsphere coupled with the anti-calprotectin detection antibody captured on T line may be corrected by the fluorescent signal (T' signal) generated by the FITC-calprotectin capture antibody conjugate coated on the T line after excitation, for example, by calculating the signal ratio: T/T' or T/(T+T'). The T signal and the T' signal were distinguished by a detector and didn't interfere with each other. The T signal and the T' signal from the same T line achieved correction on a same line. In addition, FITC in the FITC-anti-calprotectin capture antibody conjugate may be also replaced by other signal markers such as a lanthanide element or a chelate thereof, a platinum/palladium porphyrin compound, a time-resolved luminescent microsphere, a colored luminescent microsphere, a colored colloidal particle, a magnetic nanoparticle and a luminescent compound other than FITC.

The T' signal was generated by FITC in the FITC-calprotectin capture antibody conjugate, it was thus not correlated to the anti-calprotectin capture antibody in the conjugate. Furthermore, it was also possible to let FITC or a conjugate (e.g., FITC-BSA) of FITC and a carrier protein (e.g., BSA) and the anti-calprotectin capture antibody exist independently, and the two were coated on the T line.

### Embodiment 3: Manufacturing a conventional immunoassay test strip and test cassette

The conventional immunoassay test strip and test cassette manufactured in Embodiment 3 merely differed from Embodiment 1 in the following: the label pad was only coated with the time-resolved fluorescent microsphere coupled with the anti-calprotectin detection antibody, the time-resolved fluorescent microsphere coupled with DNP-BSA, and the preservation buffer solution, but was not coated with the green fluorescent microsphere coupled with the sheep anti-chicken IgY antibody; during liquid spotting on the detection pad, the T line was merely coated with the anti-calprotectin capture antibody, and not coated with the chicken IgY antibody; and the time-resolved fluorescent microsphere coupled with the calprotectin detection antibody, the time-resolved fluorescent microsphere coupled with DNP-BSA, and the preservation buffer solution were fully mixed in a ratio of 5:2:13, and then coated on the label pad. At this time, after the sample was added for reaction for a period of time, the T line only generated a detection signal (T signal) generated by the time-resolved fluorescent microsphere, but failed to generate a correction signal generated by the green fluorescent microsphere; and the time-resolved fluorescent microsphere on the C line generated a control signal (C signal). In this way, the concentration of calprotectin in the sample was thus calculated according to a signal ratio: T/C or T/(T+C).

### Embodiment 4: Detection method

The test cassette manufactured in Embodiment 1 was used to detect the calprotectin in a fecal sample, and the detailed process was as follows: 10 mg the obtained fecal sample was added to 4 ml the sample dilution solution (borax, BSA, Tween-20, EDTA-2Na, a preservative, pH 8.6), and vibrated fully and mixed to produce a turbid solution, and then 10 µl the turbid solution was taken and added to the sample inlet of the test cassette, and 80 µl the sample dilution solution was then taken and added to the sample inlet of the test cassette. After being incubated for 15 min, the test cassette was inserted into a dry-type fluorescence immunoanalyzer FIC-Q100N (Suzhou Helmen Precision Instruments Co., Ltd.) for detection.

During detection, the time-resolved fluorescent microspheres captured on the T line and C line were first excited by the excitation light with a wavelength of 360 nm generated by a light source, and then the light source was turned off; and the time-resolved fluorescent microspheres captured on the T line and C line generated time-resolved fluorescent signals with a wavelength of 615 nm. After the light source was turned off for 300 µs, the detector of the immunoanalyzer was utilized for capturing and counting the fluorescent signals to obtain the time-resolved fluorescent signal (T signal) generated on the T line and the time-resolved fluorescent signal (C signal) generated on the C line, respectively. Then, the light source generated 488 nm excitation light to excite the green fluorescent microsphere captured on the T line; after being excited, the green fluorescent microsphere captured on the T line generated a green fluorescent signal with a wavelength of 520 nm, and then the detector of the immunoanalyzer was utilized to capture the signal and the signal was counted to obtain the green fluorescent signal (T' signal) generated on the T line. To correct the adverse effect caused by the differences in the manufacturing process of a same batch of test cassettes, the T signal may be corrected in various ways. In the prior art, the T signal was usually corrected by the C signal to obtain a ratio of the T signal to the C signal, denoted as T/C, or to obtain T/(T+C). Since the C signal and the T signal were derived from the different lines, such correction was also called different-line correction. In this embodiment, the T signal was corrected by the T' signal to obtain a ratio of the T signal to the T' signal, denoted as T/T', or to obtain a ratio of T/(T+T'). Since the T signal and T' signal were derived from the same T line, such correction is also called collinear correction. Based on the calibration curve determined in advance, T/T' or T/(T+T') obtained after the addition of the clinical sample was used in determining the concentration of calprotectin.

A series of calibration solutions having different concentrations of calprotectin were prepared first, and then these calibration solutions were added to a same batch of test cassettes, respectively; after incubation for 15 min, a dry-type fluorescence immunoanalyzer was utilized to detect the T signal, T' signal, and C signal of each test cassette, and then a signal ratio: T/ T' or T/(T+T') was calculated. A calibration curve was drawn according to each signal ratio: T/ T' or T/(T+T') and its corresponding concentration of calprotectin, to calculate the concentration of calprotectin in the clinical sample during the collinear correction. As a comparison, a calibration curve was drawn according to each signal ratio: T/C or T/(T+C) and its corresponding concentration of calprotectin, to calculate the concentration of calprotectin in the clinical sample during the different-line correction.

### Embodiment 5: Detection method

The detection method in this embodiment differed from the detection method in Embodiment 4 only in the following: the test cassette manufactured by the second scheme of Embodiment 2 was used to detect calprotectin in a fecal sample; the time-resolved fluorescent microspheres captured on the T line and C line were first excited by the excitation light with a wavelength of 360 nm generated by a light source, and then the light source was turned off; a dry-type fluorescent immunoanalyzer FIC-Q100N (Suzhou Helmen Precision Instruments Co., Ltd.) was utilized to obtain the time-resolved fluorescent signal (T signal) generated on the T line and the time-resolved fluorescent signal (C signal) with a wavelength of 615 nm generated on the C line, respectively; afterwards, the T line of the test cassette was directly irradiated by the excitation light with a wavelength of 494 nm generated by the light source, and the generated light with a wavelength of 520 nm generated by the FITC-anti-calprotectin capture antibody conjugate coated on the T line was counted to obtain the fluorescent signal (T' signal) from the T line. In the test cassette manufactured in the second scheme of Embodiment 2, FITC in the FITC-calprotectin capture antibody conjugate not only acted as a quality control marker during the preparation of the test cassette, but also further generated the T' signal during detection.

### Embodiment 6: Determination of the liquid spotting uniformity on the T line

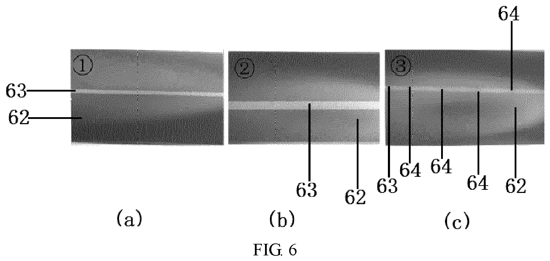

Three batches of test cassettes were manufactured according to the method in the first scheme of Embodiment 2, which differed in the following: during manufacturing the first batch of the test cassettes ①, the liquid spotting rate of the dedicated liquid scribing head was kept at 1 µl/cm; during manufacturing the second batch of the test cassettes ②, the liquid spotting rate of the dedicated liquid scribing head was increased to 2 µl/cm; and during manufacturing the third batch of the test cassettes, the liquid spotting rate of the dedicated liquid scribing head was kept at 1 µl/cm, and the membrane-spotting buffer component in the AUTOKUN continuous membrane-scribing machine/membrane-spotting machine HGS 101 was removed such that the T line solution was added discontinuously on the 2.5 cm-wide detection pads 62, thus forming a T line 63 on each 2.5 cm-wide detection pad 62. After the T line solution was added, a camera device was utilized to take pictures before drying the 2.5 cm-wide detection pads 62. The photographing results were shown in FIG 5, wherein FIG 5(a) showed a picture of the detection pads captured by the camera device after adding the T line solution on the 2.5 cm-wide detection pads during manufacturing the first batch of test cassettes, prior to the drying process of the detection pads; FIG 5(b) showed a picture of the detection pads captured by the camera device after adding the T line solution on the 2.5 cm-wide detection pads during manufacturing the second batch of test cassettes, prior to the drying process of the detection pads; FIG 5(c) showed a picture of the detection pads captured by the camera device after adding the T line solution on the 2.5 cm-wide detection pads during manufacturing the third batch of test cassettes, prior to the drying process of the detection pads. After drying the 2.5 cm-wide detection pads 62 and before cutting the test strip plate, a laser flashlight was utilized to irradiate the test strip plate such that the T lines 63 generated fluorescence; then the camera device was utilized to take pictures. The photographing results were shown in FIG 6, wherein FIG 6(a) showed a picture of the detection pads captured by the camera device after adding the T line solution, drying the 2.5 cm-wide detection pads, irradiating the test strip plate with a laser flashlight to make the T lines generate fluorescence during manufacturing the first batch of test cassettes, prior to the cutting process of the test strip plate; FIG 6(b) showed a picture of the detection pads captured by the camera device after adding the T line solution, drying the 2.5 cm-wide detection pads, irradiating the test strip plate with a laser flashlight to make the T lines generate fluorescence during manufacturing the second batch of test cassettes, prior to the cutting process of the test strip plate; FIG 6(c) showed a picture of the detection pads captured by the camera device after adding the T line solution, drying the 2.5 cm-wide detection pads, irradiating the test strip plate with a laser flashlight to make the T lines generate fluorescence during manufacturing the third batch of test cassettes, prior to the cutting process of the test strip plate. After the T line solution was added, the 2.5 cm-wide detection pads 62 were dried, and then the test strip plate was cut to obtain three batches of test cassettes (10 test cassettes were selected per batch). The method in Embodiment 2 was applied for detection to obtain the fluorescent signals on the T lines 56. The results were shown in Table 1.

**Table 1**

| No. | ① | ② | ③ | Ratio (②/①) |
|---|---|---|---|---|
| 1 | 2095 | 3739 | 1577 | 1.785 |
| 2 | 1984 | 3583 | 2258 | 1.806 |
| 3 | 1973 | 3648 | 2042 | 1.849 |
| 4 | 1989 | 3732 | 1610 | 1.876 |
| 5 | 1903 | 3777 | 1481 | 1.985 |
| 6 | 1961 | 3748 | 1553 | 1.911 |
| 7 | 1917 | 3739 | 1807 | 1.950 |
| 8 | 1961 | 3741 | 1438 | 1.908 |
| 9 | 1912 | 3854 | 1723 | 2.016 |
| 10 | 2039 | 3739 | 2066 | 1.834 |
| Mean light intensity | 1973 | 3730 | 1756 | 1.890 |
| Coefficient of variation | 3.01% | 1.93% | 15.94% | |

As demonstrated in Table 1 and FIGS. 5-6, the addition of more T line solution resulted in wider T lines on the test strip plate for manufacturing the second batch of test cassettes, and accordingly, the manufactured second batch of test cassettes had wider T lines. Continuously adding the T line solution also made the T lines on the test strip plate for manufacturing the first batch of test cassettes and the T lines on the test strip plate for manufacturing the second batch of test cassettes to be continuous, therefore, the T lines on the manufactured first and second batches of the test cassettes were also continuous and the coefficient of variations among each 10 test cassettes selected from each of the two batches of the test cassettes were small relatively, the former was 3.01% and the latter was 1.93%. The discontinuous addition of the T line solution resulted in the discontinuous T lines of the test strip plate for manufacturing the third batch of the test cassettes; that is, lots of breaking points 64 existed on the T lines of the test strip plate. The T line solution was not always absent at the breaking points 64, but the fluorescent signals at the breaking points 64 were obviously weaker than the fluorescent signals at the non-breaking points on the T lines of the test trip plate.

As the cutting positions of the T lines on the test trip plate were uncertain, the T lines on the manufactured third batch of the test cassettes may be continuous, or not continuous. Hence, 10 test cassettes were randomly selected from the third batch of the test cassettes for detection and there was a larger coefficient of variation among the 10 test cassettes, with a value of 15.94%.

The liquid spotting rate during manufacturing the second batch of test cassettes was twice the rate during manufacturing the first batch of test cassettes, and the volume of the T line solution added on the second batch of test cassettes was twice that of the first batch of test cassettes. Theoretically, the fluorescent signals generated by the T lines on the second batch of test cassettes were also twice those of the first batch of test cassettes, however, due to the limitations to the analyzer's sensitivity and other factors, there were always some deviations in practice, but it also should be approximate to 2. In terms of the 10 test cassettes selected from each batch of the test cassettes, whether for their average optical signal intensity or the optical signal intensity of each test cassette, the fluorescent signals generated by the T lines on the second batch of test cassettes were 1.785-2.016 times those of the first batch of test cassettes, and these figures were indeed approximate to 2. Therefore, according to the fluorescent signals on the T lines of the test cassettes, it was easy to determine the differences of the liquid spotting quantity and uniformity of the T line solution coated on the T lines of the test strip plate under different liquid spotting conditions.

### Embodiment 7: Detection on the influences of collinear correction on the test results

The same batch of the test cassettes manufactured in Embodiment 1 was utilized to detect the fecal samples having a low (L), moderate (M), and high (H) concentrations of calprotectin according to the detection method in Embodiment 4. Each sample was repeatedly tested for 10 times. The experimental results were shown in Table 2. These samples were subjected to the concentration detection by a calprotectin test kit (ELISA) from Buhlmann Laboratories AG The obtained detection values were as follows: the low-concentration sample: 20.57 µg/g; the moderate-concentration sample: 211.45 µg/g; and the high-concentration sample: 513.25 µg/g.

**Table 2**

| | Different-line correction (T/C) result (µg/g) | | | Collinear correction (T/T') result (µg/g) | | |
|---|---|---|---|---|---|---|
| No. | L | M | H | L | M | H |
| 1 | 25.33 | 197.53 | 565.27 | 22.52 | 213.77 | 544.95 |
| 2 | 21.58 | 184.96 | 454.40 | 25.16 | 196.91 | 607.94 |
| 3 | 21.38 | 201.13 | 459.08 | 19.44 | 230.89 | 531.55 |
| 4 | 18.49 | 246.22 | 601.92 | 19.79 | 219.99 | 483.90 |
| 5 | 19.32 | 201.04 | 435.74 | 20.45 | 201.70 | 521.12 |
| 6 | 25.84 | 222.10 | 622.33 | 22.12 | 229.05 | 544.42 |
| 7 | 20.59 | 192.35 | 472.77 | 19.10 | 207.11 | 522.44 |
| 8 | 23.44 | 214.47 | 516.78 | 21.84 | 199.62 | 526.92 |
| 9 | 16.97 | 190.55 | 525.59 | 20.66 | 228.08 | 465.41 |
| 10 | 23.36 | 215.32 | 491.09 | 22.59 | 214.25 | 546.70 |
| Coefficient of variation | 13.41% | 8.87% | 12.49% | 8.64% | 5.91% | 7.24% |

As demonstrated in Table 2, whether for the low (L), moderate (M), or high (H) calprotectin concentration fecal samples, the concentration of calprotectin in each sample may be detected accurately via the T/T'-based collinear correction. Moreover, for each sample, the coefficient of variation (CV) of the concentration values of calprotectin repeatedly detected for many times was obviously superior to that of the T/C-based different-line correction. This was because during the manufacturing of the test cassettes, the anti-calprotectin capture antibody and the chicken IgY antibody were coated on the T line for one time; even though the volume of the T line solution coated on the T line was too high or too low, the quantity of the anti-calprotectin capture antibody and the quantity of chicken IgY antibody changed synchronously. The ratio T/T' was calculated to effectively eliminate the detection difference caused by the too-high or too-low volume of the added T line solution. In comparison, the anti-calprotectin capture antibody and the rabbit anti-DNP antibody were not coated on a same line on the surface of the detection pad; the former was added on the T line, and the latter was added on the C line. Therefore, during the liquid spotting process, the quantity of the added anti-calprotectin capture antibody and the quantity of the rabbit anti-DNP antibody were hard to increase or decrease synchronously. Furthermore, because the T line and C line were separated by a certain distance; after a sample is added, the time that the sample flowed to the T line was different from that to the C line. Hence, the accumulation time of the time-resolved fluorescent microsphere on the T line was not synchronized with that on the C line. In contrast, when the sample flowed to the T line, the accumulation time of the time-resolved fluorescent microsphere on the T line was synchronized with the accumulation time of the green fluorescent microsphere on the T line. These two factors made the T/T'-based collinear correction significantly superior to the T/C-based different-line correction in detection precision.

### Embodiment 8: Detection on the influences of collinear correction on the test results

The same batch of the test cassettes manufactured in Embodiment 1 of the present invention was utilized to detect the moderate calprotectin concentration fecal samples (M) in Embodiment 7 according to the T/T'-based collinear correction method in Embodiment 4; each sample was repeatedly tested for 10 times. In the meantime, the same batch of the conventional test cassettes manufactured in Embodiment 3 were utilized to detect the moderate-calprotectin-concentration fecal samples (M) used in Embodiment 6 according to the T/C-based different-line correction method in Embodiment 4; each sample was repeatedly tested for 10 times. The experimental results were shown in Table 3.

**Table 3**

| | Different-line correction (µg/g) | Collinear correction (µg/g) |
|---|---|---|
| No. | M | M |
| 1 | 219.99 | 216.78 |
| 2 | 231.86 | 219.14 |
| 3 | 223.84 | 209.14 |
| 4 | 223.79 | 214.48 |
| 5 | 219.41 | 203.14 |
| 6 | 191.27 | 218.89 |
| 7 | 184.78 | 195.13 |
| 8 | 182.56 | 207.71 |
| 9 | 232.49 | 195.63 |
| 10 | 213.59 | 193.74 |

| | | |
|---|---|---|
| Coefficient of variation | 8.95% | 4.83% |

In this embodiment, the T/T'-based collinear correction and the T/C-based different-line correction were implemented on the different test cassettes, not on the same test cassette as in Embodiment 6. As demonstrated in Table 3, for the moderate calprotectin concentration fecal samples, the concentration of calprotectin may be detected accurately via the T/T'-based collinear correction and the CV of the calprotectin concentration values detected repeatedly for many times was obviously superior to that of the T/C-based different-line correction. Moreover, in conventional test cassettes, the label pad was not coated with the green fluorescent microsphere coupled with the sheep anti-chicken IgY antibody; the T line was not coated with the chicken IgY antibody. Hence, after adding a sample, the detection signal generated by the time-resolved fluorescent microsphere captured on the T line and the control signal generated by the time-resolved fluorescent microsphere captured on the C line were not interfered by the fluorescent signal (T') generated by the green fluorescent microsphere; but the detection precision of the T/T'-based collinear correction method was still superior to that of the T/C-based different-line correction method. This indicated that such an advantage was not achieved by the interference of the T' signal generated by the green fluorescent microsphere on the T signal and C signal, but due to the reasons mentioned in Embodiment 7.

### Embodiment 9: Detection on the influences of collinear correction on the test results

The same batch of the test cassettes manufactured in the second scheme of Embodiment 2 was utilized to detect the fecal samples having a low (L), moderate (M), and high (H) concentrations of calprotectin according to the detection method in Embodiment 5. Each sample was repeatedly tested for 10 times. The experimental results were shown in Table 4. These samples were subjected to the concentration detection by a calprotectin test kit (ELISA) from Buhlmann Laboratories AG The obtained test values are as follows: the low concentration sample: 20.57 µg/g; the moderate concentration sample: 211.45 µg/g; and the high concentration sample: 513.25 µg/g.

**Table 4**

| | Different-line correction (T/C) result (µg/g) | | | Collinear correction (T/T') result (µg/g) | | |
|---|---|---|---|---|---|---|
| No. | L | M | H | L | M | H |
| 1 | 19.54 | 202.97 | 557.29 | 19.20 | 195.13 | 574.95 |
| 2 | 19.92 | 222.63 | 531.12 | 21.97 | 185.63 | 576.72 |
| 3 | 22.89 | 207.65 | 458.55 | 20.74 | 201.31 | 659.75 |
| 4 | 20.08 | 229.74 | 608.52 | 19.95 | 190.88 | 639.51 |
| 5 | 17.11 | 254.47 | 668.67 | 18.24 | 212.56 | 638.02 |
| 6 | 15.72 | 237.36 | 452.03 | 20.87 | 215.97 | 589.74 |
| 7 | 23.02 | 298.36 | 597.25 | 22.55 | 198.08 | 599.18 |
| 8 | 14.10 | 218.86 | 526.32 | 20.64 | 210.23 | 694.87 |
| 9 | 23.30 | 258.53 | 464.75 | 22.76 | 210.83 | 556.63 |
| 10 | 22.39 | 245.86 | 542.47 | 20.36 | 197.99 | 719.13 |
| Coefficien t of variation | 13.41% | 8.87% | 12.49% | 8.64% | 5.91% | 7.24% |

As demonstrated in Table 4, whether for the low, moderate, or high calprotectin concentration fecal samples, the concentration of calprotectin in each sample may be detected accurately via the T/T'-based collinear correction. Moreover, the CV of the concentration values of calprotectin detected repeatedly for many times was obviously superior to that of the T/C-based different-line correction. One of the reasons was that in conventional test cassettes, the accuracy of the test result depended upon the amount of the time-resolved fluorescent microsphere accumulated on the T line and the amount of the time-resolved fluorescent microsphere accumulated on the C line. However, the accumulation time of the time-resolved fluorescent microsphere on the T line was not synchronized with that on the C line, which resulted in a larger fluctuation in the T/C ratio. In contrast, in the test cassette of the present invention, the amount of the FITC-anti-calprotectin capture antibody conjugate coated on the T line was fixed, which indicated that the FITC in the FITC-anti-calprotectin capture antibody conjugates was also constant. Therefore, the T' signal on the T line was unchanged and free of fluctuation, thus reducing the interference caused by the fluctuation of the T' signal.

### Embodiment 10: Detection on the influences of collinear correction on the test results

The same batch of the test cassettes manufactured in the second scheme of Embodiment 2 was utilized to detect the fecal samples having a moderate (M) calprotectin concentration used in Embodiment 7 according to the T/T'-based collinear correction method in Embodiment 5; and each sample was repeatedly tested for 10 times. In the meantime, the same batch of the conventional test cassettes manufactured in Embodiment 3 was utilized to detect the fecal samples having a moderate (M) calprotectin concentration used in Embodiment 7 according to the T/C-based different-line correction method in Embodiment 5; and each sample was repeatedly tested for 10 times. The experimental results were shown in Table 5.

**Table 5**

| | Different-line correction (µg/g) | Collinear correction (µg/g) |
|---|---|---|
| No. | M | M |
| 1 | 186.55 | 215.97 |
| 2 | 176.49 | 198.08 |
| 3 | 198.33 | 210.23 |
| 4 | 176.90 | 210.83 |
| 5 | 200.51 | 197.99 |
| 6 | 161.76 | 190.90 |
| 7 | 124.13 | 219.16 |
| 8 | 205.31 | 192.95 |
| 9 | 212.08 | 184.46 |
| 10 | 190.39 | 182.15 |
| Coefficient of variation | 14.03% | 6.54% |

In this embodiment, the T/T'-based collinear correction and the T/C-based different-line correction were implemented on different test cassettes, not on a same test cassette as in Embodiment 9. As demonstrated in Table 5, for the moderate calprotectin concentration fecal samples, the concentration of calprotectin in each sample may be detected accurately via the T/T'-based collinear correction. Moreover, the CV of the calprotectin concentration values detected repeatedly for many times was obviously superior to that of the T/C-based different-line correction.

## Claims

1. A method for detecting an analyte in a sample, comprising the following steps:
(1) providing an immunoassay test strip comprising a sample addition region and a detection pad, wherein the sample addition region is located upstream of the detection pad, the detection pad is provided with a T line, and during detection, a label reagent is located in the sample addition region or between the sample addition region and the T line and after the sample is added into the sample addition region, the label reagent moves to the T line; the label reagent comprises a first reagent marked by an analysis marker, and the first reagent is capable of specifically binding the analyte; the T line is coated with a capture reagent capable of capturing the first reagent marked by the analysis marker on the T line; a correction marker is coated on the T line, or is not coated on the T line but is capable of generating a correction signal on the T line during detection; the signals generated by the correction marker and the analysis marker are mutually distinguishable;
(2) after adding the sample into the sample addition region, the label reagent moves to the T line along with the sample and is captured by the capture reagent on the T line;
(3) utilizing an analyzer to detect a detection signal T generated by the analysis marker on the T line and a correction signal T' generated by the correction marker on the T line; and
(4) calculating a concentration of the analyte based on the signal T and the signal T'.

2. The method according to claim 1, wherein the signal T is corrected by the signal T' to obtain a corrected signal, and a concentration of the analyte is calculated based on the corrected signal.

3. The method according to claim 2, wherein the corrected signal is a signal ratio: T/T' or T/(T+T').

4. The method according to claim 1, wherein the analysis marker and the correction marker are selected from a lanthanide element or a chelate thereof, a platinum/palladium porphyrin compound, a time-resolved luminescent microsphere, a colored luminescent microsphere, a colored colloidal particle, a magnetic nanoparticle and a luminescent compound.

5. The method according to claim 1, wherein the label reagent comprises a second reagent that is marked with the correction marker, and the capture reagent is capable of capturing the second reagent marked with the correction marker on the T line.

6. The method according to claim 5, wherein the capture reagent comprises a first component and a second component, the first component is capable of capturing the first reagent marked by the analysis marker on the T line, and the second component is capable of capturing the second reagent marked by the correction marker on the T line.

7. The method according to claim 5, wherein the analysis marker is a time-resolved fluorescent microsphere, and the correction marker is a green fluorescent microsphere.

8. The method according to claim 7, wherein the second reagent/second component is selected from nonhuman antibody/anti-nonhuman antibody, biotin/streptavidin, DNP-BSA/anti-DNP antibody, and receptor/ligand.

9. The method according to claim 1, wherein the correction marker is coated on the T line, and the capture reagent comprises a first component capable of capturing the first reagent marked by the analysis marker on the T line.

10. The method according to claim 9, wherein the correction marker is coupled with the first component, or the correction marker exists independently of the first component.

11. The method according to claim 9, wherein the analysis marker is a time-resolved fluorescent microsphere, and the correction marker is fluorescein isothiocyanate(FITC) or a conjugate of FITC and a carrier protein.

12. An immunoassay test strip for detecting an analyte in a sample, comprising a sample addition region and a detection pad, the sample addition region located upstream of the detection pad, the detection pad provided with a T line, wherein during detection, a label reagent is located in the sample addition region or between the sample addition region and the T line and after the sample is added into the sample addition region, the label reagent moves to the T line; the label reagent comprises a first reagent marked by an analysis marker, and the first reagent is capable of specifically binding the analyte; the T line is coated with a capture reagent capable of capturing the first reagent marked by the analysis marker on the T line; a correction marker is coated on the T line, or is not coated on the T line but can generate a correction signal on the T line during detection; the signals generated by the correction marker and the analysis marker are mutually distinguishable.

13. The immunoassay test strip according to claim 12, wherein the analysis marker and the correction marker are selected from a lanthanide element or a chelate thereof, a platinum/palladium porphyrin compound, a time-resolved luminescent microsphere, a colored luminescent microsphere, a colored colloidal particle, a magnetic nanoparticle and a luminescent compound.

14. The immunoassay test strip according to claim 12, wherein the label reagent comprises a second reagent that is marked with the correction marker, and the capture reagent is capable of capturing the second reagent marked with the correction marker on the T line.

15. The immunoassay test strip according to claim 14, wherein the capture reagent comprises a first component and a second component, the first component is capable of capturing the first reagent marked by the analysis marker on the T line, and the second component is capable of capturing the second reagent marked by the correction marker on the T line.

16. The immunoassay test strip according to claim 14, wherein the analysis marker is a time-resolved fluorescent microsphere, and the correction marker is a green fluorescent microsphere.

17. The immunoassay test strip according to claim 12, wherein the T line is also coated with a quality control marker, and the signals generated by the quality control marker, the analysis marker and the correction marker are mutually distinguishable.

18. The immunoassay test strip according to claim 12, wherein the correction marker is coated on the T line, and the capture reagent comprises a first component capable of capturing the first reagent marked by the analysis marker.

19. The immunoassay test strip according to claim 18, wherein the correction marker is coupled with the first component, or the correction marker exists independently of the first component.

20. The immunoassay test strip according to claim 12, wherein the analysis marker is a time-resolved fluorescent microsphere, and the correction marker is FITC or a conjugate of FITC and a carrier protein.
